# EUROPEAN PATENT APPLICATION

(11) **EP 3 144 017 A1**
(43) Date of publication of application: **22.03.2017**
(21) Application number: 16189242.7
(22) Date of filing: 16.09.2016
(51) Int. Cl.: A61L 27/12, A61L 27/36, A61L 27/54

(54) **PLASMA-ENRICHED HYDROXYAPATITE-BASED FILLER MATERIAL AND METHOD OF FORMING THE SAME**

(30) Priority: 17.09.2015 US 201514857186
(71) Applicant: Metrex Research LLC, Orange, CA 92867 (US)
(72) Inventor: BURNS, Steven Joseph, Marina del Rey, CA California CA 90292 (US)
(74) Representative: Findlay, Alice Rosemary

(57) **Abstract**

Plasma-enriched hydroxyapatite-based fillers and methods of filling bone gaps in oral surgery, orthopedic procedures, and cosmetic surgery are disclosed. The fillers contain a substance comprising an anticoagulant antidote, a substance comprising hydroxyapatite, and blood or plasma. The blood may be either autologous or allogeneic. The fillers have a dough-like consistency and can be manipulated easily by a surgeon.

## Description

The present invention relates to compositions and methods for filling bone gaps. In particular, the present invention relates to hydroxyapatite-based fillers which may be used for filling bone gaps in oral surgery, orthopedic procedures, and cosmetic surgery.

During oral surgery, orthopedic procedures, and cosmetic surgery, there is often a need for bone fillers and packing materials. For instance, when synthetic implants are added to a patient's gum line, packing material or filler is used to help secure the implants by restricting motion in two dimensions. One such packing material or filler, hydroxyapatite, is a calcium phosphate commonly used as an ingredient in dental compositions, bone putties, bone cements, or as a stand-alone material, to pack around dental and orthopedic implants.

In practice, oral surgeons may mix blood, drawn from the patient during the procedure, with hydroxyapatite and, optionally, anticoagulants in an effort to include the patient's own immune system chemicals and platelets. These immune system chemicals and platelets are generally accepted as being capable of speeding osteointegration of the dental implant. The practitioner generally extracts the blood from the oral cavity just prior to the procedure. Because the blood originates from the patient undergoing the procedure, this process is autologous.

Similarly, platelet enriched plasma may be combined with the hydroxyapatite to speed osteointegration. However, the platelet enriched plasma is often, though not always, obtained from donors other than the patient, making this type of treatment an allogeneic process.

Alternatively, oral surgeons may use demineralized bone matrix materials from one or more donors, with or without hydroxyapatite, as a packing material or filler to enhance osteointegration and healing. Demineralized bone matrix contains bone morphogenic protein, a bioactive material that encourages and speeds osteointegration, and may be further combined with ground human bone allograft material. Just as with many applications of the platelet enriched plasma treatment, the addition of the demineralized bone matrix is an allogeneic process.

Each of these procedures presents certain risks and discomforts for the patient, some of which may be quite severe, and at the most extreme, can be fatal. For instance, patient blood obtained during the procedure has been in full contact with the patient's mouth, an area of the human body teeming with microorganisms. Thus, as the blood is mixed with the hydroxyapatite, the native flora adheres to the hydroxyapatite. This hydroxyapatite, blood, and microorganism mixture is then applied around the dental implant, which was previously placed in a generally sterile location of the patient's body. As a result, the patient experiences an increased risk of infection.

The use of whole blood presents further complications. For instance, whole blood contains a high percentage of red blood cells, which are not useful for osteointegration because they do not survive the process of mixing them into the hydroxyapatite. Additionally, the high concentration of red blood cells causes the patient to experience an unpleasant taste of blood during and following the procedure.

The allogeneic procedures present additional complications. First, because the tissue is derived from donors, there is an increased risk of infection transmission. Additionally, many patients experience psychological stress from knowing where the tissue derived. Also, demineralized bone matrix and platelet enriched plasma are expensive materials, a problem further exacerbated by strict rules for blood donation and good tissue practices in the United States.

Orthopedic and cosmetic surgeons perform similar procedures and face similar challenges.

In accordance with an embodiment of the invention, a bone filler is provided that includes a substance comprising an antidote to an anticoagulant (i.e., an anticoagulant antidote); a substance comprising hydroxyapatite; and blood or plasma. The bone filler does not include bone tissue.

In accordance with another embodiment of the invention, a method of forming a bone filler is provided. The method includes mixing together a substance comprising an anticoagulant antidote, a substance comprising hydroxyapatite, and blood or plasma to form the bone filler.

Also disclosed is a method of filling bone gaps. The method includes adding a substance comprising an anticoagulant antidote and a substance comprising hydroxyapatite to the bone gap. Blood or plasma is added to the bone gap to form the bone filler. No bone tissue is added to the bone filler.

The invention will now be further described by way of examples.

In accordance with embodiments of the present invention, a bone filler is provided that promotes osteointegration of synthetic implants, such as titanium implant anchors and implant bone screws. The bone filler includes a substance comprising an anticoagulant antidote, a substance comprising hydroxyapatite, and blood or plasma. The bone filler does not include bone tissue.

In the discussion that follows, all weight percentages are based on the total weight of the specified composition, unless stated otherwise.

In accordance with embodiments of the present invention, the substance comprising an anticoagulant antidote includes the antidote *per se*. The anticoagulant antidote, or clotting accelerator, may include protamine sulfate and salts, manganese sulfate (MnSO₄), magnesium sulfate (MgSO₄) and its hydrates, calcium chloride (CaCl₂), any soluble calcium or lithium salt, or thrombin. Of course, the enumerated antidotes are exemplary, not limiting, and one of ordinary skill in the art is capable of selecting an appropriate antidote in view of the particular application for which the bone filler is to be compounded.

In accordance with certain additional embodiments of the present invention, the substance comprising hydroxyapatite may contain not only hydroxyapatite *per se*, but also certain excipients, such as thickeners, diluents, pH buffers, acids and bases to help control pH, adhesives, polymeric compounds, and fluorescing compounds. When present, the excipients may form 5 % (weight/weight) or less of the dry weight composition.

A number of thickeners are known. For instance, the thickener may be a synthetic thickener, such as sodium alginate, propylene glycol alginate, sodium carboxymethyl cellulose, calcium carboxymethyl cellulose, sodium carboxymethyl starch, sodium starch phosphate, sodium polyacrylate, methyl cellulose, hydroxypropyl cellulose, or polyvinylpyrrolidone The thickener may also be a natural thickener, such as cyamoposis gum, Carob bean gum, Tara gum, Tamarind seed gum, gum arabic, tragacanth gum, Karaya gum, alginic acid, carrageenan, xanthan gum, gellan gum, curdlan, chitin, chitosan, chitosamine, or Irish moss. Alternatively, the thickener may be an inorganic thickener such as calcium carbonate, calcium silicate, silica powder, amorphous hydrous silicate, silica aerogels, hydrophobic silica, or various precipitated silicas. These thickeners can be used alone or in any combination thereof.

The diluents may be any diluents known to those of ordinary skill in the art, such as starch, cellulose and its derivatives, magnesium stearate, polyethylene glycol, and esters of mellitic acid. These diluents can be used alone or in any combination thereof.

The pH of the substance comprising hydroxyapatite, and ultimately, that of the bone filler may be controlled by the addition of one or more buffers, one or more acids, one or more bases, or any combination thereof. Possible buffers are not particularly limited. For instance, the buffer may be any buffer approved for use by the United States Food & Drug Administration ("US FDA"), such as acetate, citrate, phosphate, borate, or carbonate buffer systems, or a mixed buffer system. Similarly, possible acids and bases are not particularly limited and may, for instance, be any acid or base approved for use by the US FDA. Such acids may include any organic or inorganic acid, such as acetic acid, small quantities of hydrochloric acid, citric acid, and phosphoric acid. Such bases may include carbonates, bicarbonates, hydroxides, and ammonia.

In addition, the substance comprising hydroxyapatite may include one or more adhesives or polymeric materials. For instance, cyanoacrylate-based adhesives enjoy widespread use among oral surgeons, although other adhesives and polymeric materials may be included. Adhesives and polymeric substances may include 1,6-hexanediyl bismethacrylate, 2-hydroxy-1,3-propanediyl bismethacrylate, 7,7,9(or 7,9,9)-trimethyl-4,13-dioxo-3,14-dioxa- 5, 12-diazahexadecane-1, 16-diyl bismethacrylate, 3-trimethoxysilylpropyl methacrylate, and 1,1,3,3-tetramethylbutyl hydroperoxide. Peroxides may serve as polymerization initiators. In some embodiments, a chemical polymerization initiator, if required, may be physically separated from the polymer in a tube, container, or skimmer. In other embodiments, the polymerization may be activated by water in the blood plasma.

The substance comprising hydroxyapatite may include one or more fluorescent compounds. Exemplary fluorescent compounds include fluorescein (D&C Yellow no. 7) and/or its disodium salt uranine (D&C Yellow no. 8). Including such fluorescent compounds in the formulation allows the oral surgeon to confirm proper placement of the filler material, using an ultraviolet light source, for example.

Any number of these optional excipients may be included in the bone filler. The enumerated thickeners, diluents, pH buffers, acids, bases, adhesives, polymeric compounds, and fluorescing compounds are exemplary and not limiting. One of ordinary skill in the art is capable of selecting the appropriate excipients for the particular application for which the bone filler is to be compounded.

The bone filler also includes blood or plasma. The blood or plasma may be obtained from the patient just prior to the procedure. Autologous blood or plasma mixed with hydroxyapatite creates a custom filler optimized for the patient receiving treatment with respect to biocompatibility and immunocompatibility. Alternatively, the blood or plasma may be obtained from a donor, although an allogeneic source of blood or plasma introduces risks associated with disease transmission, blood type cross-match errors, and unpredictable immunological reactions.

The blood may be obtained by conventional venipuncture techniques, rather than extracting blood from the oral cavity. Venipuncture is a procedure where small volumes of blood are extracted, usually in an evacuated tube, through a cannulated venous needle, which provides a sterile procedure, thereby reducing the chance for an infection to develop. Many sizes of these tubes are commercially available and can be preloaded with an optional anticoagulant, if desired. Additionally, surgeons and medical staff are well-versed in venipuncture techniques. Similarly, the blood may be collected in standard tubes containing separators, e.g. a separator gel. Thus, the patient's blood may be extracted several days prior to the oral surgery for which the filler is formulated and then centrifuged to separate red blood cells from plasma. Prior blood extraction may be preferred, for example, if the oral surgeon wishes not to collect the necessary blood in the office, but instead prefers to have the blood collected at an offsite clinic.

If an anticoagulant is included in the bone filler, the anticoagulant may be any anticoagulant known in the art. For instance, the anticoagulant may be a heparin salt, an EDTA (Ethylenediaminetetraacetic acid) salt, or a citrate salt. One of ordinary skill in the art is capable of selecting the appropriate anticoagulant for the particular application for which the bone filler is to be compounded.

A method of filling bone gaps is provided which includes mixing a substance comprising an anticoagulant antidote, a substance comprising hydroxyapatite, and blood or plasma. The mixture is then applied to the bone gaps.

The substance comprising hydroxyapatite may be sterilized prior to its use using any sterilization methods known in the art. For instance, the substance comprising hydroxyapatite may be sterilized by heat sterilization in which saturated steam under pressure is applied to the substance in an autoclave. Alternatively, the substance may be sterilized by a dry-heat method in which the substance is exposed to heat for an extended period of time. Alternatively, the substance may be sterilized by exposure to ionizing radiation, such as gamma radiation from cobalt-60, at a dose sufficient to render it sterile. Other sterilization techniques are known, and one of ordinary skill in the art is capable of determining an appropriate sterilization technique in view of the substance being sterilized, including any excipients contained in that substance.

As noted above, the blood or plasma may originate from the patient or from a donor. In certain embodiments of the present invention, plasma is added to the substance comprising the anticoagulant antidote and the substance comprising hydroxyapatite prior to applying the mixture to the bone gap. The plasma may be pre-centrifuged from the whole blood of the patient or donor.

In accordance with certain embodiments of the invention, the substance comprising hydroxyapatite and the substance comprising the anticoagulant antidote are mixed in a chamber. Then, previously-centrifuged plasma is added to the chamber. Upon contact, the mixture begins to clot and a doughy mix results. This doughy mix is then applied to the bone gap, taking advantage of the malleable consistency of the doughy mix.

In accordance with other embodiments of the invention, a sample of whole blood in a chamber preloaded with one or more anticoagulants is spun in a centrifuge until the red blood cells settle at the bottom of the chamber and the plasma settles on top of the red blood cells. A serum skimmer preloaded with the substance comprising hydroxyapatite and the substance comprising the anticoagulant antidote is added to the chamber and pushed to the interface between the plasma and red blood cells. On contact, the anticoagulant antidote contained in the serum skimmer and the anticoagulants contained in the plasma react to form a doughy mix. As in embodiments previously described, this doughy mix is then applied to the bone gap, taking advantage of the malleable consistency of the doughy mix.

In accordance with yet further embodiments of the present invention, the substance comprising hydroxyapatite, anticoagulant, and the substance comprising the anticoagulant antidote are mixed in a chamber. A sample of whole blood is added to the chamber. The entire mixture is then spun in a centrifuge until the red blood cells settle at the bottom of the chamber and a mixture of plasma, anticoagulant, and the substance comprising the anticoagulant antidote settles on top of the red blood cells. The anticoagulant and anticoagulant antidote interact, producing a doughy mix. The doughy mix is then applied to the bone gap, taking advantage of the malleable consistency of the doughy mix.

A further method of filling bone gaps is provided which includes applying a substance comprising an anticoagulant antidote and a substance comprising hydroxyapatite to the bone gap. Then plasma or blood is added to the bone gap. This mixture then thickens in the bone gap.

Without intending to be bound by any particular theory, it is believed that bone fillers formed in accordance with the present invention include fibrin, cofactors, and platelets that are active and, therefore, effective at promoting osteointegration. As such, the anticoagulant antidote inactivates the anticoagulant, when present, to begin a clotting process. As clotting develops in the plasma, fibrin forms, aiding with adhesion at the surgical site and thickening the bone filler to a useful consistency. If placed into the surgical site soon after mixing, the bone filler will actually complete clotting in-situ, exactly molded to the shape of the implant and associated bone gap.

Alternatively, when no anticoagulant is included, it is believed that the calcium and rough micro-surface of the hydroxyapatite particles initiate the clotting process immediately. If whole blood is used in such a system, the bone filler could be used as is or could be spun in a centrifuge to remove much of the red blood cells, which migrate to the bottom of the centrifuge tube. Scraping away the top fraction provides the bone filler. However, because no anticoagulant is present, the clotting process operates more quickly, and a bone filler produced in accordance with this embodiment of the present invention should be used as soon as possible after centrifugation.

The advantageous properties of this invention can be observed by reference to the following examples, which illustrate but do not limit the invention.

### EXAMPLES

Exemplary formulations of embodiments of the present invention are provided in Table 1. To prepare the formulations, the anticoagulant antidote was added to a solution of blood and the anticoagulant in the amounts provided in Table 1. In the table, the amount of anticoagulant in the blood is given per ml of blood, and the amount of anticoagulant antidote is given per ml of final composition.

**Table 1**

| Example | Anticoagulant | Anticoagulant dose in blood | Anticoagulant antidote | Antidote dose in final composition |
|---|---|---|---|---|
| 1 | Lithium heparin | 10 - 30 USP units | Protamine sulfate | 20 - 400 µg |
| 2 | Sodium heparin | 10 - 30 USP units | Protamine sulfate | 20 - 400 µg |
| 3 | Potassium EDTA | 1-2mg | Calcium chloride | 1.5 - 25 mg |
| 4 | Potassium EDTA | 1-2mg | Calcium chloride | 26 - 100 mg |
| 5 | Sodium EDTA | 1-2mg | Calcium chloride | 1.5 - 25 mg |
| 6 | Sodium EDTA | 1-2mg | Calcium chloride | 26 - 100 mg |
| 7 | Sodium citrate / citric acid buffer | 3-4mg | Calcium chloride | 7 - 25 mg |
| 8 | Sodium citrate / citric acid buffer | 3-4mg | Calcium chloride | 25 - 50 mg |
| 9 | Sodium citrate / citric acid buffer | 3-4mg | Thrombin | ≥ 2 units |

The time required for gelling the formulations of certain embodiments of the invention may be controlled by modifying the anticoagulant antidote concentration, as shown in Table 2. To prepare the formulations provided in Table 2, heparinized calf's blood was mixed with protamine sulfate. This mixture was then poured into dry hydroxyapatite, which was previously roughly ground, to form a test solution that remained in a liquid state for more than 5 minutes. In Example 10, 250 microliters of a 2.4 mg/mL solution of protamine sulfate in physiological saline was added to the heparinized blood to a total volume of 5 mL, to give 120 micrograms of protamine sulfate per ml of solution. The solution was placed into a water bath heated to 34.5 °C and allowed to set. When checked at 3 minutes, the liquid had already gelled into a semi-solid substance. After gelling, the material was still malleable but no longer liquid. In Examples 11-13, lower concentrations of protamine sulfate were mixed and the above experimental procedure was repeated until a working time of 5 minutes was observed.

**Table 2**

| Example | Amount of protamine sulfate (µl of 2.4 mg/ml solution in physiological saline) | Concentration of protamine sulfate | Gel time (min.) |
|---|---|---|---|
| 10 | 250 | 120 | <3 |
| 11 | 220 | 106 | 3 |
| 12 | 200 | 96 | 4 |
| 13 | 170 | 82 | 6 |

Thus, the concentration of the protamine sulfate can be used to tailor the working time for the filler material, depending on the procedure being performed. Similar results were obtained with anticoagulant antidotes other than protamine sulfate. See Table 1, and in particular, the ranges provided for the anticoagulant antidotes. Stated differently, these results demonstrate that the amount of anticoagulant antidote added may be decreased to create a longer working time, in those situations where such a longer working time would be desired, and may be increased to create a shorter working time, in those situations where such a shorter working time is desired.

While the present invention has been illustrated by the description of one or more embodiments thereof, and while the embodiments have been described in considerable detail, additional advantages and modifications will readily appear to those skilled in the art.

## Claims

1. A bone filler, comprising:
a first substance comprising an anticoagulant antidote;
a second substance comprising hydroxyapatite; and
blood or plasma;
wherein the bone filler is free of bone tissue.

2. The bone filler of claim 1, wherein the bone filler further comprises an anticoagulant.

3. The bone filler of claim 2, wherein the anticoagulant is a heparin salt, an EDTA salt, a citrate salt, or a mixture thereof.

4. The bone filler of any preceding claim, wherein the first substance includes one or more excipients selected from the group consisting of thickeners, diluents, pH buffers, acids and bases, adhesives, polymeric compounds, and fluorescing compounds.

5. The bone filler of any preceding claim, wherein the anticoagulant antidote is selected from the group consisting of protamine sulfate, salts of protamine sulfate, calcium chloride, soluble calcium or lithium salts, thrombin, and mixtures thereof.

6. The bone filler of any preceding claim, wherein the blood or plasma is autologous.

7. A method of forming a bone filler comprising:
mixing together a substance comprising an anticoagulant antidote, a substance comprising hydroxyapatite, and blood or plasma to form the bone filler wherein the bone filler is free of bone tissue.

8. The method of claim 7, wherein the substance comprising hydroxyapatite is sterilized prior to the mixing.

9. The method of either claim 7 or claim 8, wherein the mixing comprises:
first mixing the substance comprising hydroxyapatite and the substance comprising the anticoagulant antidote in a chamber; and
then adding plasma to the chamber to mix with the substance comprising hydroxyapatite and the substance comprising the anticoagulant antidote to form the bone filler.

10. The method of claim 7, said method comprising
separating red blood cells from plasma of the blood; and
contacting a mixture of the substance comprising the anticoagulant antidote and the substance comprising hydroxyapatite with the plasma to form the bone filler.

11. The method of claim 10, wherein separating red blood cells from plasma is performed by centrifugation to give a red blood cell fraction and a plasma fraction.

12. The method of claim 11, wherein contacting the mixture of the substance comprising the anticoagulant antidote and the substance comprising hydroxyapatite with the plasma is performed by
loading a serum skimmer with the substance comprising the anticoagulant antidote and the substance comprising hydroxyapatite; and
adding the serum skimmer to the plasma fraction.

13. The method of claim 7, said method comprising
mixing together the substance comprising the anticoagulant antidote, the substance comprising hydroxyapatite, and an anticoagulant to form a first mixture;
adding whole blood to the first mixture to form a second mixture;
separating red blood cells from the second mixture to form a red blood cell fraction and a third mixture fraction; and
isolating the third mixture fraction to form the bone filler.

14. The method of any one of claims 7 to 13, wherein the substance comprising hydroxyapatite further comprises an anticoagulant.

15. The method of claim 14, wherein the anticoagulant is a heparin salt, an EDTA salt, a citrate salt, or mixtures thereof.
